(19) Europäisches Patentamt / European Patent Office / Office européen des brevets

(11) **EP 3 355 058 B1**

(12) **EUROPEAN PATENT SPECIFICATION**

(45) Date of publication and mention of the grant of the patent: **25.12.2019 Bulletin 2019/52**

(51) Int Cl.: ***G01N 33/68*** *(2006.01)*

(21) Application number: **17153745.9**

(22) Date of filing: **30.01.2017**

(54) **NEW TEST FOR MONITORING RENAL DISEASE**

NEUER TEST ZUR ÜBERWACHUNG VON NIERENERKRANKUNG

NOUVEAU TEST DE SURVEILLANCE DE MALADIE RÉNALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application: **01.08.2018 Bulletin 2018/31**

(73) Proprietor: **Helena Laboratories (UK) Ltd.**
**Gateshead, Tyne and Wear NE11 0SD (GB)**

(72) Inventors:
- **Delanghe, Sigurd**
  **9000 Gent (BE)**
- **Moerman, Alena**
  **9000 Gent (BE)**
- **Speeckaert, Marijn**
  **9000 Gent (BE)**
- **Delanghe, Joris**
  **9000 Gent (BE)**

(74) Representative: **V.O.**
**P.O. Box 87930**
**Carnegieplein 5**
**2508 DH Den Haag (NL)**

(56) References cited:
**EP-A1- 1 229 325**
**WO-A1-97/11363**
**WO-A2-2013/010085**

- **CHRISTIANE DRECHSLER ET AL: "Protein carbamylation is associated with heart failure and mortality in diabetic patients with end-stage renal disease", KIDNEY INTERNATIONAL, vol. 87, no. 6, 1 June 2015 (2015-06-01), pages 1201-1208, XP55371883, LONDON, GB ISSN: 0085-2538, DOI: 10.1038/ki.2014.429**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 355 058 B1

## Description

The invention relates to a new method for determining carbamylated serum albumin in a blood or plasma sample and thereby a new method to monitor disease development that is characterized by an increase in urea, more particularly diseases like chronic kidney disease (CKD) and other renal diseases, such as ESRD (end-stage renal disease).

Chronic kidney disease (CKD) is estimated to affect 5-10% of adults in industrialized countries, such as the United States (Eknoyan et al., Kidney Int. 66: 1310-4, 2004). All patients with evidence of persisting kidney damage, i.e. for >90 days, are defined as having CKD. Kidney damage refers to any renal pathology that has the potential to cause a reduction in renal functional capacity. This is most usually associated with a reduction in glomerular filtration rate (GFR) but other important functions may be lost without this occurring.

CKD normally is classified in sub-stages on basis of the amount of GFR reduction according to the following table:

Table 1. Stratification of chronic kidney disease

| Stage | Description | GFR (ml/min/1.73 $m^2$) |
|---|---|---|
| 1 | Kidney damage with normal or raised GFR | ≥ 90 |
| 2 | Kidney damage with mild decrease in GFR | 60 - 89 |
| 3A | Moderately lowered GFR | 45 - 50 |
| 3B | | 30 - 44 |
| 4 | Severely lowered GFR | 15 - 29 |
| 5 | Kidney failure (end-stage renal disease) | < 15 |

Kidney damage may be detected either directly or indirectly. Direct evidence may be found on imaging or on histopathological examination of a renal biopsy. A range of imaging modalities including ultrasound, computed tomography (CT), magnetic resonance imaging (MRI) and isotope scanning can detect a number of structural abnormalities including polycystic kidney disease, reflux nephropathy, chronic pyelonephritis and renovascular disease.

Renal biopsy histopathology is most useful in defining underlying glomerular disease such as immunoglobulin A (IgA) nephropathy or focal glomerulosclerosis.

Indirect evidence for kidney damage may be inferred from analysis of urine or blood. Glomerular inflammation or abnormal function can lead to leakage of red blood cells or protein into the urine which in turn may be detected as proteinuria or haematuria. Urinary abnormalities may have alternative causes unrelated to kidney dysfunction and there are methodological issues associated with their measurement.

The clinical evidence on urine tests suggests that urine dipstick testing cannot reliably be used to diagnose the presence or absence of proteinuria although there is evidence that dipstick proteinuria (≥1+) predicts ESRD and cardiovascular disease. There is no evidence that isolated asymptomatic UTI causes proteinuria/albuminuria. Protein/creatinine ratio (PCR) and albumin/creatininen ratio (ACR) are more accurate rule-out tests in populations with a high probability of proteinuria. PCR and ACR predict subsequent progression of renal disease. ACR has also been shown to predict cardiovascular disease, although similar evidence for PCR was not identified.

Historically, measurement of creatinine or urea in serum or plasma has been used to assess kidney function. Both are convenient but insensitive (glomerular filtration rate has to halve before a significant rise in serum creatinine becomes apparent). In addition, serum concentrations of creatinine are
affected by various analytical interferences, and depend critically on muscle mass, for example, a serum creatinine concentration of 130 micromol/l might be normal in one individual but requires further investigation in another.

Various other markers have been used to estimate clearance, including inulin, iohexol and radioisotopic markers such as 51Cr-ethylenediaminetetraacetic acid (EDTA), 99mTc-diethylenetriaminepentaacetic acid (DTPA) and 125I-iothalamate. Measurement of any of these markers is too costly and labour intensive to be widely applied. For the purposes of evaluating methods of GFR assessment, inulin clearance is widely regarded as the most accurate (gold standard) estimate of GFR, whilst the radioisotopic methods listed above are accepted as validated reference standards

Carbamylation is the nonenzymatic binding of urea-derived cyanate to free amino groups on proteins, and it accumulates as kidney function declines (Kwan, J. et al., 1992, Ann. Clin. Biochem. 29:206-209; Balion, C. et al., 1998, Kidney Int. 53:488-495)). The post-translational protein alterations of carbamylation have been implicated in the progression of various diseases by changing the charge, structure, and function of enzymes, hormones, and receptors (e.g. Jaisson, S. et al., 2011, Clin. Chem. 57:1499-1505). For example, when carbamylated, proteins as diverse as collagen and LDL accelerate the biochemical events of atherosclerosis. Proteins with long half-lives provide a time-averaged indication of carbamylation burden analogous to the relationship between serum glucose and glycated hemoglobin (Kwan, 1992).

The proportion of carbamylated albumin is strongly correlated with blood urea concentrations and strongly associated with all-cause mortality in hemodialysis patients (Ber, A. et al., 2013, Sci. Transl. Med. 5:175ra29). In this study it was found that the proportion of carbamylated human albumin, which has a predominant carbamylation site on Lys(549) (%C-Alb) correlated with time-averaged blood urea concentrations and was twice as high in ESRD patients than in non-uremic subjects (0.90% versus 0.42%). It was suggested that measurement of carbamylated human albumin would be a good indicator for the progress of CKD, especially ESRD (US20140228296).

Standard methods may be used to measure levels of carbamylated albumin in any bodily fluid, including, but not limited to, urine, serum, plasma, saliva, amniotic fluid, or cerebrospinal fluid. Such methods include immunoassay, enzymatic immunoassay, ELISA, "sandwich assays," immunoturbidimetry, immunonephelometry, western blotting using antibodies specifically directed to carbamylated albumin, immunodiffusion assays, agglutination assays, fluorescent immunoassays, protein A or G immunoassays, and immunoelectrophoresis assays and quantitative enzyme immunoassay techniques such as those described in Ong et al. (Obstet. Gynecol. 98:608-11, 2001) and Su et al. (Obstet. Gynecol. 97:898-904, 2001). Most of these methods, however are quite laborious and/or quite costly and a simpler, cheaper method is desired.

At present, liquid chromatography assessment of carbamylation requires analysis of characteristic carbamylation-derived compounds such as e-carbamyllysine (homocitrulline) (Jaisson S. et al. Anal Bioanal Chem 2012;402:1635-41) .

Capillary electrophoresis is available in most routine clinical laboratories. The technique is characterized by a high resolution, which makes is well suited to detect posttranscriptional changes. Albumin is the most abundant plasma protein and has an in vivo half-life of 10.5 $\pm$ 1.5 days (Sterling, K., J Clin Invest. 1951;30(11):1228-1237.), which makes it well suited for studying carbamylation.

The present inventors now found that capillary electrophoresis can be very well used to assess the amount of carbamylated albumin and by doing this to diagnose and follow the development of chronic kidney disease.

Accordingly, the invention comprises a method for monitoring renal disease in a subject, comprising subjecting a serum sample comprising albumin from that subject to electrophoresis, preferably capillary electrophoresis, more preferably wherein the capillary is a fused silica capillary, wherein carbamylated albumin is used as a bio-marker for the monitoring of the renal disease. Preferably in said method the electrophoresis is carried out in a buffered aqueous liquid, having an alkaline pH. Further details about preferred buffers can be found below. Further, it is preferred that the sample is blood serum.

The invention is specifically advantageous when the renal disease is ESRD.

In a further preferred embodiment the monitoring comprises determining the symmetry ratio of the serum albumin peak is determined (which ratio is a measure for the relative amount of carbamylated serum albumin with respect to native serum albumin).

Further part of the invention is the use of electrophoresis, preferably capillary electrophoresis, using a separation buffer having a pH in the range of 9.3 to 10.7 or a separation buffer, comprising borate, having a pH in the range of 8 to 11 .to determine the absolute or relative amount of carbamylated serum albumin in a biological fluid, based on the symmetry ratio of the serum albumin peak..

Also part of the present invention is a method for determining the relative amount of carbamylated serum albumin with respect to native serum albumin via capillary electrophoresis on basis of the symmetry ratio of the electrophoresis serum albumin peak.

In another embodiment the invention comprises a method to assess the severity of renal disease by determining the symmetry ratio of the serum albumin peak (representing carbamylated and non-carbamylated albumin) in an electrophoresis assay, preferably a capillary electrophoresis assay.

The invention further comprises a system for the determination of carbamylated serum albumin, said system comprising:

- an electrophoresis system, preferably a capillary electrophoresis system;
- computation means adapted to compute the symmetry of the electrophoresis serum albumin peak;
- output means for delivering the output of the calculation and/or the determination.

Preferably said system is a system wherein the computation means comprise a computer provided with appropriate software algorithms. Further preferred is a system, wherein the output means comprise an output screen, printer or any other device for communication with the user of the system. In another preferred embodiment the system is a portable system. In a further embodiment the system further comprises a generator for an alarm signal that is triggered if the symmetry ratio reaches a predetermined threshold value

LEGENDS TO THE FIGURES

Fig.1 shows *in vitro*-carbamylation of serum albumin, in the upper left corner a control specimen, right upper corner following incubation with ImMol/L KCNO (symmetry-factor: 0,648), left below with 10 mMol/L KCNO (symmetry factor: 0,576) and right below following an incubation with 150 mmol/L KCNO.

Fig. 2: Spectrum following capillary electrophoresis of serum showing the calculation of the symmetry factor. The x-axis depicts the migration time (s), the y-axis was normalized on the albumin peak.

Fig. 3. Symmetry factor of serum albumin in controls and CKD stages (1-5) (n = 240).

Fig. 4: ROC curve of the albumin symmetry factor for CKD stage 2 till 5. The y-axis depicts the sensitivity, the x-axis the aspecificity.

DETAILED DESCRIPTION

Definitions

All technical and scientific terms used herein have the same meaning as commonly understood by one of ordinary skill in the art to which this invention belongs, unless the technical or scientific term is defined differently herein.

The term "or" as used herein means "and/or" unless specified otherwise. The term "a" or "an" as used herein means "at least one" unless specified otherwise.

When referring to a noun (e.g. a compound, an additive, etc.) in singular, the plural is meant to be included.

When referring herein to a pH, the pH as measured by a Mettler-Toledo SevenEasy meter with an InLab Expert Pro open junction electrode with integral Argenthal reference electrode and temperature sensor, calibrated by a three-point calibration curve with reference pHs of 4.00, 7.00 and 10.00 at 20 °C is meant, unless specified otherwise.

The "symmetry ratio" of a peak is a measure for the skewedness of a peak obtained in the electrophoresis, preferably capillary electrophoresis. A peak that has a symmetry ratio of 1 is a peak of which the two halves, when separated by a vertical line at the top of the peak are completely symmetrical., i.e. when those two halve are exact mirror images. The symmetry ratio is less than one if one slope of the peak is more skewed than the other slope. More precisely (see also Fig. 2), at the Y-value of the albumin peak, corresponding to 10% of the maximal height of the albumin peak, the horizontal distance from the left point of the curve to the line perpendicular to the top of the peak is designated as "a", the distance from the corresponding point on the right part of the curve to that perpendicular line is designated "b". The symmetry factor is formed by the ratio a/b.

Electrophoresis has been a well-established method for analysing various samples, including samples comprising biological compounds such as proteins, for many decades.

Over the years well-established methods have been developed. For instance, the separation of serum proteins in a series of characteristic bands, commonly referred to as gamma, beta, alpha-1 and 2 and albumin by electrophoresis is well known to those skilled in the art and is well described in a wide range of reference materials, such as the handbook 'Protein Electrophoresis in Clinical Diagnosis' by David F. Keren, 2003, Arnold Publications.

Capillary electrophoresis is a specific form of electrophoresis, wherein a capillary is used to perform the electrophoresis. Capillary electrophoresis offers advantages such as short analysis times and a high resolution.

In principle any form of electrophoresis may be used. In particular, good results have been obtained with zone electrophoresis.

Preferably, the electrophoresis is carried out in a channel, although in principle a method according to the invention can be carried out on a plate or the like (as in conventional flat gel electrophoresis). In a particularly preferred embodiment, the channel is a capillary, in which case the electrophoresis technique is generally referred to as capillary electrophoresis (CE). In a specific embodiment, electrophoresis may be carried out in a channel of a microfluidic device or the like, which technique is often referred to in the art as 'CE on a chip'. More information on CE may be found in the review paper 'Clinical Analysis by Microchip Capillary Electrophoresis' by Sam Li and Larry Kricka, Clinical Chemistry 52, p37-45, 2006, and the references cited therein.

The inner wall of the channel (capillary), may in particular comprise acidic groups of which at least a portion dissociate when in contact with the alkaline buffer, such that use can be made of an electro-osmotic flow during the separation. In a particularly preferred method of the invention using capillary electrophoresis, the electro-osmotic flow is directed from the inlet side of the capillary to the outlet side of the capillary, whereby also sample constituents (such as proteins) that have the same sign of charge as the outlet electrode (negative charge) are dragged towards the detector, and thus are detected Suitable materials are generally known in the art. A much preferred material comprising such acidic groups is silica, in particular fused silica.

- The use of blood plasma or blood serum as a sample is particularly advantageous because the pH variation in such samples is relatively small, e.g. compared to urine. Also the sample to sample variation in the concentrations of the major electrolytes is relatively small between various blood plasma/serum samples. This is advantageous in particular, because changes in pH or electrolyte concentration in a sample can affect the shape of the protein peaks, such as the albumin peak. Thereby, accurate determination of a deviation from a normal peak shape, and thus from a deviation in the symmetry ratio..

The separation conditions may be based on a method known *per se*, *e.g.* in the above described prior art for the analysis of a specific analyte, in this case serum albumin and/or carbamylated serum albumin. Also buffer solutions for electrophoresis are commercially available, for various analytes, especially for serum proteins, such as serum albumin.

In general it is advantageous to use a buffer solution having a pH that is about the same as or higher than the (average) pKa of the analyte, such that at least the majority of any acid functions on this molecule are ionised to form anionic groups. In particular, the separating of the sample may be carried out using a solution having an alkaline pH, in particular a pH in the range of pH 8.0 to 11.0, preferably a pH in the range of pH 9.0 to 10.7, more in the range of 9.3-10.7, e.g. about 10.0. Preferably, the solution comprises a pH-buffer. Such buffer is in general a combination of at least one acid and at least one base (which may be the conjugated base of the acid), with a pKa of about the pH of the solution (generally the pKa being in the range of pH +/- 1 pH unit, preferably in the range of +/- 0.5 pH units).

Examples of acid/bases that may be used to provide buffer solutions are borate, phosphate and carbonate buffers, buffers based on amino acids and zwitterionic compounds for providing buffers, known as biological buffers. Examples of acids/bases for biological buffers include bis-TRIS (2-bis[2-hydroxyethyl]amino-2-hydroxymethyl-1,3-propanediol), ADA (N-[2-acetamido]-2-iminodiacetic acid), ACES (2-[2-acetamino[-2-aminoethanesulphonic acid), PIPES (1,4-piperazinediethanesulphonic acid), MOPSO (3-[N-morpholino]-2-hydroxypropanesulphonic acid), bis-TRIS PROPANE (1,3-bis[tris(hydroxymethyl)methylaminopropane]), BES (N,N-bis[2-hydroxyethyl]-2-aminoethanesulphonic acid), MOPS (3-[N-motpholino]propancsulphonic acid), TES (2-[2-hydroxy-1,1-bis(hydroxymethyl)ethylamino]ethanesulphonic acid), HEPES (N-[2-hydroxyethyl]piperazine-N'-(2-ethanesulphonic)acid), DIPSO (3-N,N-bis[2-hydroxyethyl]amino-2-hydroxypropanesulphonic acid), MOBS (4-N-morpholinobutanesulphouic acid), TAPSO (3[N-tris-hydroxymethyl-methylamino]-2-hydroxypropanesulphonic acid), TRIS (2-amino-2-[hydroxymethyl]-1,3-propanediol), HEPPSO (N-[2-hydroxyethyl]piperazine-N'-[2-hydroxypropanesulphonic]acid), POPSO (piperazie-N,N'-bis[2-hydroxypropanesulphonic]acid), TEA (triethanolamine), EPPS (N-[2-hydroxyethyl]-piperazine-N'-[3-propanesulphonic]acid), TRICINE (N-tris[hydroxymethyl]methylglycine), GLY-GLY (diglycine), BICINE (N,N-bis[2-hydroxyethyl]glycine), HEPBS (N-[2-hydroxyethyl]piperazine-N'-[4-butanesulphonic]acid), TAPS (N-tris[hydroxymethyl]methyl-3-aminopropanesulphonic acid), AMPD (2-amino-2-methyl-1,3-propanediol), TABS (N-tris[hydroxymethyl]methyl-4-aminobutanesulphonic acid), AMPSO (3-[(1,1-dimethyl-2-hydroxyethyl)amino]-2-hydroxypropanesulphonic acid), CHES (2-(N-cyclohexylamino)ethanesulphonic acid), CAPSO (3-[cyclohexylamino]-2-hydroxy-1-propanesulphonic acid), AMP (2-amino-2-methyl-1-propanol), CAPS (3-cyclohexylamino-1-propanesulphonic acid) and CABS (4-[cyclohexylamino]-1-butanesulphonic acid).

Suitable kits comprising a buffer and instructions are commercially available, e.g. CE-Sure SPE kit, available since the mid 1990's, from Helena Biosciences (Gateshead, UK). Furthermore, in US 2002/0162744, which describes an additive that interacts with at least one serum protein, in particular albumin, and modifies it's electrophoretic mobility, an alkaline buffer solution of pH 10 is described.

When a serum or blood sample is subjected to electrophoresis peaks will appear in the output at instances where (high) concentrations of proteins are present in the sample. The by far largest peak in the electrophoresis output of a blood or serum sample is the peak that belongs to albumin, and this peak this is always recognizable. It now has appeared that the shape of the albumin peak changes as a result of carbamylation . In particular, a slope of this peak becomes more skewed as the fraction of carbamylated albumin increases. It now has appeared that there is a more or less dose-dependent relation with the amount of carbamylation of the albumin proteins (which in itself is related to the severity of the renal disease) and the skewedness of the albumin peak. The skewedness of the albumin peak can easily be calculated as the symmetry ratio of the peak. It has appeared that the symmetry ratio of the albumin peak in a sample of a healthy person was about 0.75 - 0.95, while the symmetry ratio of a sample of a person with ESDR was about 0.4 - 0.6. More particularly, when KCNO was added to a sample containing albumin to an amount of 10 mM already a clear distinction with the non-treated sample could be established. It was further found that it was even possible to use the symmetry ratio parameter to distinguish between the different stages of renal disease as define above. These values are based on analyses with the CE buffer for serum protein analysis as can be commercially obtained from Helena Biosciences, Tyne and Wear, United Kingdom.

Accordingly, the present invention may be used to monitor the development of renal disease in a subject. Such a monitoring comprises a repetitive measurement which will indicate whether the amount of carbamylation and with that the severity of the disease has increased or decreased. Such a monitoring can advantageously be used to investigate whether a treatment of the disease would have had success and/or whether the condition of the patient ameliorates.

When comparing capillary electrophoresis to HPLC it appears that CE has a better resolution, reduced sample preparation, faster analysis, reduced reagent consumption and reduced cost. Further, only sample volumes in the pico- and nanoliter range are needed. As compared to mass spectroscopy, CE is a very simple, fast and cheap method.

Another advantage of CE is the absence of fronting: in HPLC fronting may occur when the column capacity is exceeded. Capillary electrophoresis combines high throughput and sensitive analyses. Moreover, the technique can be automated. Also, separation is extremely efficient, as evidenced by theoretical plate numbers exceeding 10,000 (Bortolotti F. et al., Clin Chim Acta. 2007;380:4-7).

In albumin, the carbamylation-reaction preferentially takes place on lysine residue 549. Lysine residues carry a positive charge at physiological pH. Attaching a $-CONH_2$ group induces a loss of this positive charge. Albumin, which is negatively charged at physiological pH migrates during electrophoresis towards the positive electrode because the electroosmotic flow outweighs the electrophoretic mobility. The elimination of a positive charge associated with the formation of ε-carbamyl-lysine of homocitrulline increases the electrophoretic mobility, resulting in a slower migration. Fronting is partially due to the more slowly migration of albumin (Petersen, C.E., J Biol Chem 2000, 275(28): 20985-95).

It is also possible to use the electrophoretic analysis as a diagnostic instrument to indicated the severity stage of renal disease (see also Figure 3 and Table 2 in the experimental part).

Further, the detection of the symmetry ratio of the albumin peak also enables a quantitative detection of the amount of carbamylated albumin in the sample. First of all, a quantification of the percentage carbamylation may be directly obtained from the symmetry ratio. And, of course, if the exact amount of non-carbamylated albumin or of total albumin in the sample is known, the exact amount of carbamylated albumin can then be calculated

The electrophoresis analysis is preferably performed in a system in which the electrophoresis analyser is coupled to computation means to compute the symmetry of the electrophoresis serum albumin peak and output means for delivering the output of the calculation and/or the determination.

Such computation means may be any computer or device comprising a software algorithm which computer or device is capable to connect to and receive the output of the electrophoresis analyser, is capable to detect the peak that belongs to albumin and is capable of measuring the skewedness of said peak and from that the symmetry ratio as defined above. In this system, the output of the electrophoresis analyser may be analog or digital. If the output is analog, the computation means generally will comprise an analog-to-digital converter in order to digitize the input and make it suitable for further analysis and detection by the software algorithm. This software algorithm (also indicated herein as "program") is able to detect the peak that belongs to albumin. This can advantageously be done to detect the peak with the highest value in the complete electrophoresis signal. Then the program should calculate the are under the curve for the peak (AUC) and from there the skewedness of the slopes of the peak. It will then derive a figure indicating the symmetry ratio. This figure can be shown on the output means, but the computation means may also 'translate' the result obtained to a renal disease state (according to table shown above) of compare the result obtained to earlier measurements from the same subject. In order to make comparisons, the computation means should have access to a memory storage where the results of earlier measurements of the same subject should be obtainable and in order for the system to know which sample is measured, there should be input means on which the nature (and identification) of the subject can be indicated and any further details that need to be stored with the results of the sample. Of course, if results need to be stored, the computation means should have a connection to a suitable storage medium, such as a hard disk or any other computer data storage device. It would also be possible that the data is stored at a location remote from the system. For this to be accomplished, the system should be able to connect to this remote storage medium either by an intermittent direct coupling (regularly 'hooking up' to the storage device) or through a wireless connection, such as infrared, WiFi (3G, 4G, 5G) or BlueTooth.

The output means may be any means on which an output can be visualised, such a by print, sound or by screen. Accordingly, the output means may be a printer or a display, such as a LED screen or any other kind of display.

In an embodiment, the device is a device that is portable. This may advantageously be reached if the electrophoresis analyser itself is small, such as a microfluid device or chip. A microfluid device has the further advantage that the volume of the sample that needs to be provided can be very small. Therefore, it has also become practical that the sample can be taken without an intervention by a doctor or other medically educated personnel. For instance, if the sample may be small it would be very practical and advantageous by obtaining a sample through a finger prick. Then, the subject itself would be able to obtain a sample and conduct the test.

In such a case, a preferred embodiment of the present invention is a system which is capable of providing an alarm signal if the measured value exceeds a pre-set threshold value. The alarm can take any form, such as a sound or light signal and/or a text message on the output device of the system. If the system is able to connect to remote systems (such as discussed for storage of data above), it can also be advantageous if the alarm signal is sent to such a remote location. In this way, it may be possible that the measured data and the alarm message is transmitted to and/or stored in the system of the physician or medical specialist where the subject is under treatment.

The invention will now be illustrated by the following examples.

EXAMPLE

## Materials and methods

### Study population

Thirty nine healthy volunteers (20 men, 19 women, age: 39 ± 12 years) participated in the study. The reference range was defined by the interval between percentiles 2.5 and 97.5 of the control group. Furthermore, serum was investigated of 20 chronic kidney disease patients presenting with CKD 2 (12 men, 8 women), 21 patients showing stages CKD 3A (16 men, 5 women) and 3B (15 men, 6 women), 21 patients presenting with CKD stage 4 (15 men, 6 women). Furthermore, 73 end stage renal disease (CKD 5) patients (47 men, 26 women), treated with chronic hemodialysis, were enrolled in the study.

The study was approved by the local ethics committee (2015/0932, Belgian registration number B670201525559)

Blood was drawn, tubes were centrifuged (10 min, 3000 rpm, room temperature) and serum was obtained. In ESRD patients, blood was sampled before the start of a hemodialysis session. Routine parameters urea, creatinine, uric acid, and albumin were determined in serum. Albumin was assayed using immunonephelometry on a BN II Nephelometer (Siemens). Creatinine was assayed using a compensated rate-blanked picrate assay (Wuyts, B. et al., Clin Chem. 2003;49: 1011-4). Urea was assayed on a Modular analyzer (Roche).

eGFr was estimated using the CKD-EPI 2009 (chronic kidney disease epidemiology collaboration) formula (Levey, A.S. et al., Ann Intern Med. 2009;150(9):604-612).

*In vitro* carbamylation of serum was achieved by adding 50 μL potassium cyanate (Sigma, St Louis) in phosphate buffered saline (0.1 mol/L, pH 7.4) to 500 μL of serum. Solutions with final concentrations of 10 to 100 mMol/L were used.

Standardized Kt/V (stdKT/V) was calculated according to the formula (Gotch, F.A. et al., Kidney Int 2000, 58(76): 3-18).:

$$std\frac{Kt}{V} = 7.24.60.60.\frac{m}{C_0.V}$$

in which K stands for urea clearance ($m^3/s$), t for dialysis time (s), V for the distribution volume ($m^3$), m for the mass generation rate of urea (mol/s) and Co for the urea concentration at the start of dialysis ($mol/m^3$).

Capillary electrophoresis of serum was carried out using a Helena V8 capillary electrophoresis system (Helena, Newcastle, UK). Two μL of each serum sample was 50-fold diluted (0.9% saline) and was automatically injected. Separation of proteins was obtained by applying a voltage of 9 kV in eight fused-silica capillaries controlled by Peltier effect. A ninth capillary was used as reference (buffer only). In the standard operating mode, direct detection of proteins was performed by measuring UV absorbance of the separated fractions at 210 nm. Throughput was 90 samples/h. Helena V8 CE was used in a standard setting for serum electrophoresis (Poisson, J. et al. Clin Biochem 2012;45:697-9, 12).

Following electrophoresis, morphology of albumin peaks was analyzed by calculating the asymmetry factor, making use of the Platinum 4.1® software. The asymmetry factor is a measure of peak tailing. It is defined as the distance from the center line of the peak to the back slope divided by the distance from the center line of the peak to the front slope, with all measurements made at 10% of the maximum peak height.

The reproducibility of the assay was evaluated by repeating a series (n =10) of three different serum pools showing respectively pronounced, average and little fronting.

Statistics

Statistical analysis was carried out using the program MedCalc. Normality of disibutions was tested using the D'Agostino Pearson test. Differences between patient groups were assessed using the Kruskall-Wallis test. To investigate the correlation between 2 non-normal continuous variables, a rank correlation test was carried whereby Spearman's rho was calculated.

For studying the effect of the various parameters on the albumin symmetry factor as dependent variable, a multiple regression model was used. A backward method was used. Variables were included in the model if p-values were lower than 0,05. Based on the pooled dataset, an ROC (Receiver operating characteristic) curve was constructed.

## Results

In vitro-carbamylation (final isocyanate concentration: 10 -100 mmol/L) resulted in a dose-dependent alteration of the albumin peak symmetry. Figure 1 depicts the pherograms of uncarbamylated and carbamylated serum and shows the gradual change in albumin peak morphology.

Reproducibility of the assay . Within-run coefficient of variation was 1.1 %. Linearity was observed in the concentration range.

The reference range for the albumin symmetry factor was found to be 0.69 -0.92.

*In vivo*-carbamylated serum samples showed similar changes. Figure 3 and Table 1 compare the symmetry of the albumin peaks between controls and the various CKD stages.

**Table 2: Depiction of median, maximum and minimum of the symmetry factor in healthy controls and in various stages of chronic kidney disease**

| | Median | Maximum | Minimum |
|---|---|---|---|
| CONTROLS (n = 39) | 0,824 | 0,921 | 0,683 |
| CKD 2 (n = 20) | 0,807 | 0,904 | 0,628 |
| CKD 3A (n = 21) | 0,751 | 0,845 | 0,679 |
| CKD 3B (n = 21) | 0,723 | 0,813 | 0,553 |
| CKD 4 (n = 21) | 0,676 | 0,914 | 0,530 |
| Hemodialysis patients UZ GENT (n = 73) | 0,644 | 0,880 | 0,345 |
| Hemodialysis patients AALST (n = 47) | 0,602 | 0,822 | 0,342 |

In the overall group, albumin peak asymmetry correlated well with average serum urea concentration (y (symmetry factor; %) = -0.001948 x (average serum urea concentration) + 0.828; r = 0.575, p <0.0001). A similar correlation was found with serum creatinine, (y (symmetry factor; %) = - 0.02569 x (average serum creatinine concentration) -0.0257; r = 0.652, p <0.0001).

Correlation with KT/V was rather poor: (y (symmetry factor; %) = - 0.061 x (stdKT/V) +0.416; r = 0.242, n.s.). stdKT/V is generally regarded as a poor marker for assessing dialysis efficiency

Receiver operating characteristics (ROC) analysis showed an area under curve (AUC) of 0.916 (95 % CI: 0.873 - 0.948) for the diagnosis of CKD stage 2.

## Claims

1. Method for monitoring renal disease in a subject, comprising subjecting a serum sample comprising albumin from that subject to electrophoresis, wherein carbamylated albumin is used as a bio-marker for the monitoring of the renal disease, wherein the monitoring comprises determining the symmetry ratio of the electrophoresis serum albumin peak.

2. Method to assess the severity of renal disease by determining the symmetry ratio of the serum albumin peak in an electrophoresis assay,.

3. Method according to any of the preceding claims, wherein the renal disease is end-stage renal disease.

4. Method for determining the relative amount of carbamylated serum albumin with respect to native serum albumin via electrophoresis, on basis of the symmetry ratio of the electrophoresis serum albumin peak.

5. Method according to any of the preceding claims, wherein said electrophoresis is carried out using a separation buffer comprising borate, said buffer having a pH in the range of 8 to 11.

6. Method according to any of the preceding claims, wherein said electrophoresis is carried out using a separation buffer having a pH in the range of 9.3 to 10.7.

7. Method according to any of the preceding claims, wherein the sample is a blood serum sample.

8. Method according to any of the preceding claims, wherein the electrophoresis is capillary electrophoresis.

9. Method according to claim 8, wherein the measurement takes place in a fused silica capillary.

10. Use of electrophoresis, preferably capillary electrophoresis, more preferably capillary electrophoresis using a separation buffer as defined in claim 5 or 6, to determine the absolute or relative amount of carbamylated serum albumin in a biological fluid, based on the symmetry ratio of the serum albumin peak.

11. System for the determination of carbamylated serum albumin, said system comprising:

- an electrophoresis system, preferably a capillary electrophoresis system;
- computation means adapted to compute the symmetry of the electrophoresis serum albumin peak;
- output means for delivering the output of the calculation and/or the determination of carbamylated serum albumin based on the symmetry ratio.

12. System according to claim 11, wherein the computation means comprise a computer provided with appropriate software algorithms.

13. System according to claim 11 or 12, wherein the output means comprise an output screen, printer or any other device for communication with the user of the system.

14. System according to any of claims 11- 13, wherein the system is a portable system.

15. System according to any of claims 11 - 14, wherein the system further comprises a generator for an alarm signal that is triggered if the symmetry ratio reaches a predetermined threshold value.

## Patentansprüche

1. Verfahren zum Überwachen von Nierenerkrankung bei einem Subjekt, umfassend Unterziehen einer Serumprobe, umfassend Albumin, von dem Subjekt einer Elektrophorese, wobei carbamyliertes Albumin als ein Biomarker für das Überwachen der Nierenerkrankung verwendet wird, wobei das Überwachen Bestimmen des Symmetrieverhältnisses des Elektrophorese-Serumalbuminpeaks umfasst.

2. Verfahren, um die Schwere einer Nierenerkrankung durch Bestimmen des Symmetrieverhältnisses des Serumalbuminpeaks in einem Elektrophorese-Assay zu beurteilen.

3. Verfahren nach einem der vorhergehenden Ansprüche, wobei die Nierenerkrankung eine Endstadium Nierenerkrankung ist.

4. Verfahren zum Bestimmen der relativen Menge an carbamyliertem Serumalbumin in Bezug auf natives Serumalbumin mittels Elektrophorese, auf Basis des Symmetrieverhältnis des Elektrophorese-Serumalbuminpeaks.

5. Verfahren nach einem der vorhergehenden Ansprüche, wobei die Elektrophorese unter Verwendung eines Trennpuffers, umfassend Borat, durchgeführt wird, der Puffer mit einen pH in dem Bereich von 8 bis 11.

6. Verfahren nach einem der vorhergehenden Ansprüche, wobei die Elektrophorese unter Verwendung eines Trennpuffers mit einem pH in dem Bereich von 9,3 bis 10,7 durchgeführt wird.

7. Verfahren nach einem der vorhergehenden Ansprüche, wobei die Probe eine Blutserumprobe ist.

8. Verfahren nach einem der vorhergehenden Ansprüche, wobei die Elektrophorese Kapillarelektrophorese ist.

9. Verfahren nach Anspruch 8, wobei die Messung in einer Quarzglaskapillare erfolgt.

10. Verwendung von Elektrophorese, vorzugsweise Kapillarelektrophorese, bevorzugter Kapillarelektrophorese unter Verwendung eines Trennpuffers, wie definiert in Anspruch 5 oder 6, um die absolute oder relative Menge von carbamyliertem Serumalbumin in einer biologischen Flüssigkeit, basierend auf dem Symmetrieverhältnis des Serumalbuminpeaks, zu bestimmen.

**11.** System für die Bestimmung von carbamyliertem Serumalbumin, das System umfassend:

- ein Elektrophoresesystem, vorzugsweise ein Kapillarelektrophoresesystem;
- Berechnungsmittel, angepasst, um die Symmetrie des Elektrophorese-Serumalbuminpeaks zu berechnen;
- Ausgabemittel zum Liefern der Ausgabe der Berechnung und/oder der Bestimmung von carbamyliertem Serumalbumin, basierend auf dem Symmetrieverhältnis.

**12.** System nach Anspruch 11, wobei die Berechnungsmittel einen Computer, bereitgestellt mit geeigneten Softwarealgorithmen, umfassen.

**13.** System nach Anspruch 11 oder 12, wobei das Ausgabemittel einen Ausgabebildschirm, Drucker oder ein anderes Gerät zur Kommunikation mit dem Benutzer des Systems umfassen.

**14.** System nach einem der Ansprüche 11 - 13, wobei das System ein tragbares System ist.

**15.** System nach einem der Ansprüche 11 - 14, wobei das System ferner einen Generator für ein Alarmsignal umfasst, das ausgelöst wird, wenn das Symmetrieverhältnis einen vorbestimmten Schwellenwert erreicht.

## Revendications

**1.** Procédé de surveillance d'une maladie rénale chez un sujet, comprenant la soumission d'un échantillon de sérum comprenant de l'albumine provenant de ce sujet à une électrophorèse, dans lequel de l'albumine carbamylée est utilisée comme biomarqueur pour la surveillance de la maladie rénale, dans lequel la surveillance comprend la détermination du rapport de symétrie du pic d'albumine sérique lors de l'électrophorèse.

**2.** Procédé d'évaluation de la gravité de l'insuffisance rénale par détermination du rapport de symétrie du pic d'albumine sérique dans un essai d'électrophorèse.

**3.** Procédé selon l'une quelconque des revendications précédentes, dans lequel l'insuffisance rénale est une insuffisance rénale terminale.

**4.** Procédé pour la détermination de la quantité relative d'albumine sérique carbamylée par rapport à l'albumine sérique native par électrophorèse, sur la base du rapport de symétrie du pic de l'albumine sérique lors de l'électrophorèse.

**5.** Procédé selon l'une quelconque des revendications précédentes, dans lequel ladite électrophorèse est réalisée en utilisant un tampon de séparation comprenant du borate, ledit tampon ayant un pH compris entre 8 et 11.

**6.** Procédé selon l'une quelconque des revendications précédentes, dans lequel ladite électrophorèse est réalisée en utilisant un tampon de séparation ayant un pH compris entre 9,3 et 10,7.

**7.** Procédé selon l'une quelconque des revendications précédentes, dans lequel l'échantillon est un échantillon de sérum sanguin.

**8.** Procédé selon l'une quelconque des revendications précédentes, dans lequel l'électrophorèse est une électrophorèse capillaire.

**9.** Procédé selon la revendication 8, dans lequel la mesure est effectuée dans un capillaire en silice fondue.

**10.** Utilisation de l'électrophorèse, de préférence de l'électrophorèse capillaire, plus préférentiellement de l'électrophorèse capillaire utilisant un tampon de séparation tel que défini dans la revendication 5 ou 6, pour déterminer la quantité absolue ou relative d'albumine sérique carbamylée dans un fluide biologique, sur la base du rapport de symétrie du pic de l'albumine sérique .

**11.** Système pour la détermination de l'albumine sérique carbamylée, ledit système comprenant:

- un système d'électrophorèse, de préférence un système d'électrophorèse capillaire;
- des moyens de calcul adaptés pour calculer la symétrie du pic d'albumine sérique d'électrophorèse;
- des moyens de sortie pour délivrer la sortie du calcul et/ou la détermination de l'albumine sérique carbamylée en fonction du rapport de symétrie.

**12.** Système selon la revendication 11, dans lequel les moyens de calcul comprennent un ordinateur pourvu d'algorithmes logiciels appropriés.

**13.** Système selon la revendication 11 ou 12, dans lequel les moyens de sortie comprennent un écran de sortie, une imprimante ou tout autre dispositif pour communiquer avec l'utilisateur du système.

**14.** Système selon l'une quelconque des revendications 11 à 13, dans lequel le système est un système portable.

**15.** Système selon l'une quelconque des revendications 11 à 14, dans lequel le système comprend en outre un générateur pour un signal d'alarme qui est déclenché si le rapport de symétrie atteint une valeur seuil prédéterminée.

**Fig. 1**

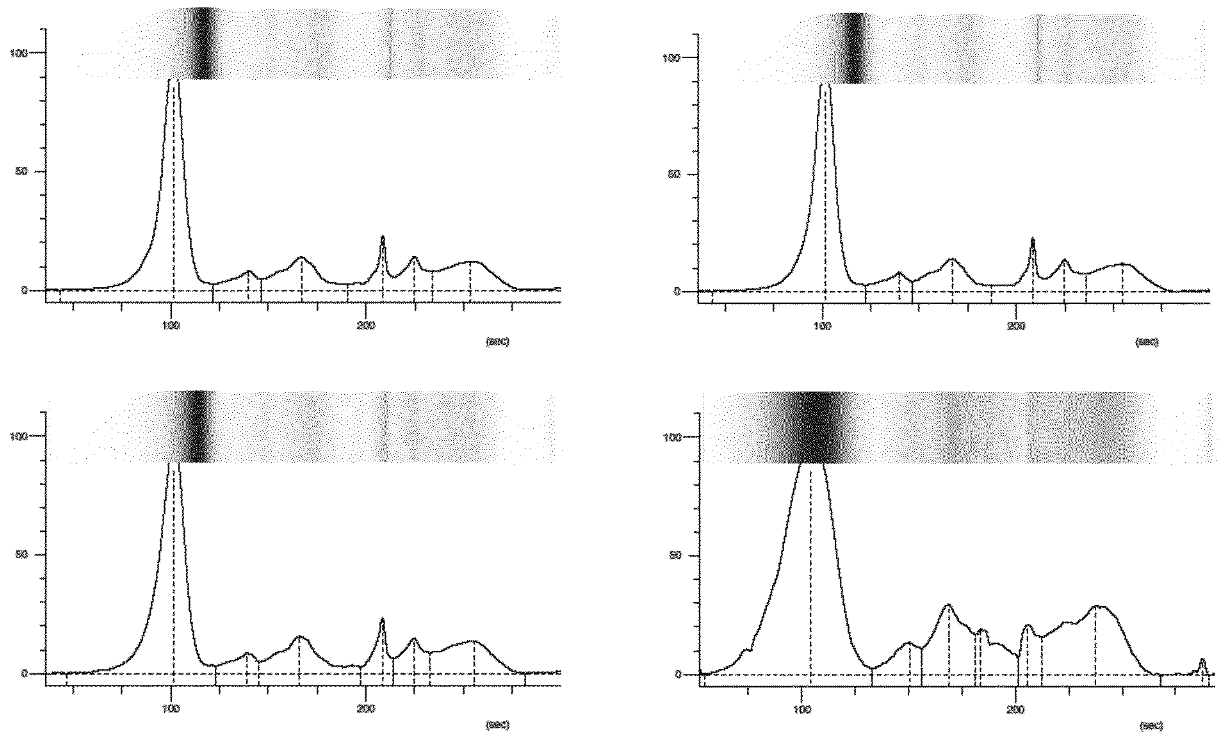
100
50
0
100
200
(sec)

**Fig. 2**

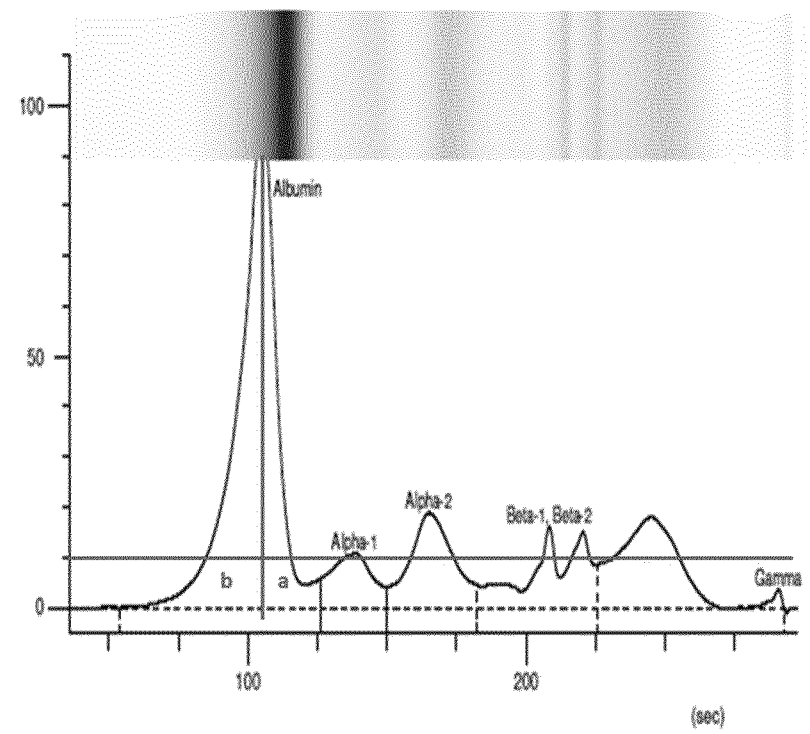
100
50
0
Albumin
Alpha-1
Alpha-2
Beta-1, Beta-2
Gamma
b
a
100
200
(sec)

**Fig. 3**

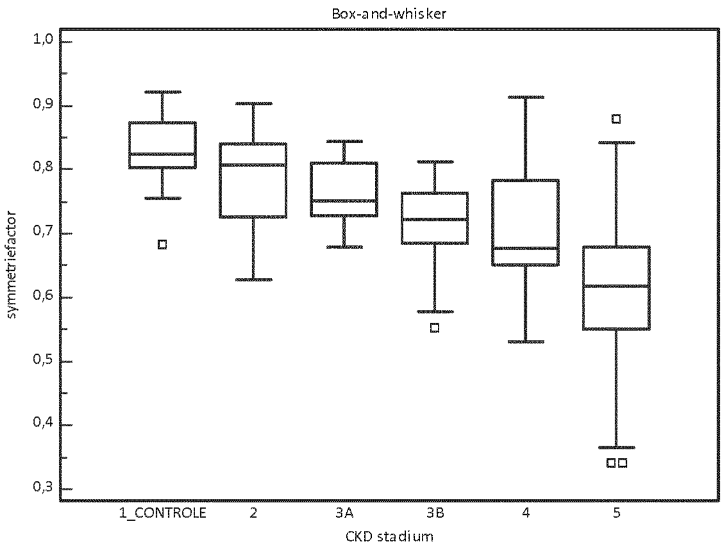
Box-and-whisker
1,0
0,9
0,8
0,7
0,6
0,5
0,4
0,3
symmetriefactor
1_CONTROLE
2
3A
3B
4
5
CKD stadium

**Fig. 4**

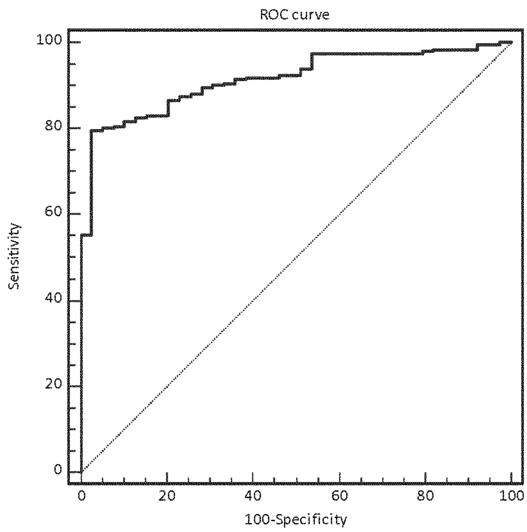
ROC curve
100
80
60
40
20
0
Sensitivity
0
20
40
60
80
100
100-Specificity

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20140228296 A
- US 20020162744 A

### Non-patent literature cited in the description


- **EKNOYAN et al.** *Kidney Int.,* 2004, vol. 66, 1310-4
- **KWAN, J. et al.** *Ann. Clin. Biochem.,* 1992, vol. 29, 206-209
- **BALION, C. et al.** *Kidney Int.,* 1998, vol. 53, 488-495
- **JAISSON, S. et al.** *Clin. Chem.,* 2011, vol. 57, 1499-1505
- **BER, A. et al.** *Sci. Transl. Med.,* 2013, vol. 5, 175ra29
- **ONG et al.** *Obstet. Gynecol.,* 2001, vol. 98, 608-11
- **SU et al.** *Obstet. Gynecol.,* 2001, vol. 97, 898-904
- **JAISSON S. et al.** *Anal Bioanal Chem,* 2012, vol. 402, 1635-41
- **STERLING, K.** *J Clin Invest.,* 1951, vol. 30 (11), 1228-1237
- **DAVID F. KEREN.** Protein Electrophoresis in Clinical Diagnosis. Arnold Publications, 2003
- **SAM LI ; LARRY KRICKA.** Clinical Analysis by Microchip Capillary Electrophoresis. *Clinical Chemistry,* 2006, vol. 52, 37-45
- **BORTOLOTTI F. et al.** *Clin Chim Acta.,* 2007, vol. 380, 4-7
- **PETERSEN, C.E.** *J Biol Chem,* 2000, vol. 275 (28), 20985-95
- **WUYTS, B. et al.** *Clin Chem.,* 2003, vol. 49, 1011-4
- **LEVEY, A.S. et al.** *Ann Intern Med.,* 2009, vol. 150 (9), 604-612
- **GOTCH, F.A. et al.** *Kidney Int,* 2000, vol. 58 (76), 3-18
- **POISSON, J. et al.** *Clin Biochem,* 2012, vol. 45, 697-9, 12